# EUROPEAN PATENT SPECIFICATION

(11) **EP 2 183 380 B1**
(45) Date of publication and mention of the grant of the patent: **26.11.2014**
(21) Application number: 08798774.9
(22) Date of filing: 27.08.2008
(51) Int. Cl.: C12N 9/12, C12P 19/34

(54) **METHODS AND COMPOSITIONS FOR PCR**
VERFAHREN UND ZUSAMMENSETZUNGEN FÜR PCR
PROCÉDÉS ET COMPOSITIONS POUR UNE PCR

(30) Priority: 27.08.2007 US 968196 P
(43) Date of publication of application: 12.05.2010
(73) Proprietor: Life Technologies Corporation, Carlsbad, CA 92008 (US)
(72) Inventor: XI, Larry, L., Foster City, CA (US); PROSEN, Dennis, E., Foster City, CA (US); NOVIKOV, Alexander, A., Foster City, CA (US)
(74) Representative: Grund, Martin
(86) International application number: PCT/US2008/074432
(87) International publication number: WO 2009/029648

(56) References cited:
- US-A- 5 677 152
- US-A- 5 677 152
- US-A1- 2006 228 726
- US-A1- 2006 228 726

## Description

### FIELD

This invention relates to compositions and methods for reversible inactivation of thermostable DNA polymerases.

### INTRODUCTION

Non-specific amplification has long plagued the application of the Polymerase Chain Reaction (PCR) and substantial resources have been expended to minimize this problem (see e.g. Chou et al., Nucleic Acids Research, 20(7): 1717-1723 (1992)). Several "hot-start" PCR techniques have been used to overcome difficulties related to non-specific amplification products caused by the extension of mis-primed oligonucleotides during reaction set-up or the initial heating phase of PCR. In one method, an essential PCR component such as the oligonucleotide primers, nucleotide triphosphates, magnesium ions or thermostable nucleic acid polymerase is added only at higher temperatures, thereby reducing the probability of having non-specific hybridization or extending mis-primed oligonucleotides. In another method, described in U.S. Pat. No. 5,411,876, a solid wax-barrier is used between the template-primer mix and the remaining reaction mixture. This wax-barrier melts only at elevated temperature, so that all of the reaction components are mixed only at high temperature, preventing mis-priming and extension of mis-primed oligonucleotides. Another method uses a compound that binds specifically to single-stranded DNAs (primers) in a heat-reversible manner, such as a single-strand binding protein, to prevent extension of mis-primed oligonucleotides, as disclosed in U.S. Pat. App. No. 2006/0029952A1. Reversible modifications, both non-covalent and covalent, of the nucleic acid polymerase, have also been used. U.S. Pat. No. 5,338,671 discloses the use of antibodies specific for the nucleic acid polymerase to inhibit the polymerase's activity. However, this method carries the risk of contamination due to an increased number of handling steps. U.S. Pat. No. 5,677,152, to Birch et al. ("the Birch '152 Patent") describes a PCR method using a chemically or covalently modified thermostable polymerase. Specifically, the DNA polymerase is reversibly inactivated using a dicarboxylic acid anhydride, and the thermostable DNA polymerase can be activated after incubation for a certain period of time at elevated temperature. Finally, U.S. Pat. No 6,183,998 discloses reversible inactivation of thermostable polymerase using an aldehyde, preferably formaldehyde.

Apart from non-specific amplification due to mis-priming, another area of concern in nucleic acid amplification and cloning is the fidelity of the DNA polymerases (see e.g. Hengen, 1995. Methods and reagents - Fidelity of DNA polymerases for PCR. Trends in Biochemical Sciences 20 (8): 324-325). Because of the low fidelity of the Taq DNA polymerase, several thermostable Pol B enzymes have been developed and marketed, including Pfu, Vent@, Deep Vent@, KOD, Phusion™, iProof™, Tli and Pfx. Different from the Taq DNA polymerase, the residual polymerase activity of Family B DNA polymerase (Pol B) at temperatures below 50 °C is small, leading some manufacturers of these enzymes to claim that they are "natural" hot-start DNA polymerases for PCR. However, these enzymes exhibit a substantial 3'-5' nuclease activity at room temperature, causing significant primer degradation and alteration of the specificity of the primers. The degradation of the primers may lead to mis-priming and/or formation of primer-dimers during PCR carried later on. For example, some manufactures warn the users that the 3'-5' exonuclease activity of their products must be well controlled to achieve desired results. One of the methods to control the 3'-5' exonuclease activities is to use two antibodies to bind to the enzyme, one for the DNA polymerase activity and one for 3'-5' nuclease activity. See e.g. Mizuguchi et al. J Biochem (Tokyo). 1999, 126:762-8. The same group also demonstrated that KOD with excessively active proofreading activity at temperatures where PCR was carried out was not beneficial to PCR and a mutant that has a lowered proofreading activity became a better PCR-enzyme

(Kuroita et al., Structural mechanism for coordination of proofreading and polymerase activities in archaeal DNA polymerases. J Mol Biol. 2005, 351:291-8). A commercial product alone this line is also available (the KOD DNA polymerases by Takara Shuzo Co., Ltd., Japan). As indicated above, however, antibody binding of the enzymes entails the risks of contamination. In addition, the antibodies are expensive and difficult to procure.

Accordingly, there is a need for methods and compositions that overcome the shortcomings of existing thermostable DNA polymerases.

### SUMMARY

Disclosed is a modified thermostable Pol B DNA polymerase having both DNA polymerase and 3'-5' exonuclease activities, wherein the modified thermostable Pol B DNA polymerase is produced by reacting, under essentially aqueous conditions, a thermostable Pol B DNA polymerase with a modifier reagent of Formula I 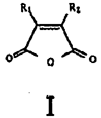 wherein R₁ and R₂ are hydrogen or a C₁-C₄ alkyl which may be linked, wherein the reaction results in inactivation of the thermostable Pol B DNA polymerase activity and the 3'-5' exonuclease activity, and wherein the thermostable Pol B DNA polymerase activity and the 3'-5' exonuclease activity are restorable.

Accordingly, the present invention provides a modified thermostable Pol B DNA polymerase having both a DNA polymetase activity and a 3'-5' exonuclease activity, wherein the modified thermostable Pol B DNA polymerase is produced by reacting, under essentially aqueous conditions, a thermostable Pol B DNA polymerase with a modifier reagent of Formula I 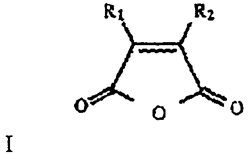 wherein R₁ and R₂ are hydrogen or a C₁-C₄ alkyl which may be linked,
wherein the reaction results in inactivation of the thermostable Pol B DNA polymerase activity and the 3'-5' exonuclease activity, wherein a ratio between the polymerase activity and the 3'-5' exonuclease activity is restorable to a value that is essentially the same as before modification, wherein the thermostable Pol B DNA polymerase activity and the 3'-5' exonuclease activity are restorable by incubating the thermostable Pol B DNA polymerase at an elevated temperature that is > 50 °C, and wherein the Pol B DNA polymerase is a fusion protein which comprises Pfu, KOD, TIi, or Pfx, or a DNA polymerase sold under the trade names Vent®, Deep Vent@, Phusion™, or iProof™, or a fragment thereof having DNA polymerase activity and 3'-5' exonuclease activity and a nucleic acid binding polypeptide.

Also provided is the above modified thermostable Pol B DNA polymerase, wherein the Pol B DNA polymerase is 10His-Pfu-Pae3192.

Also provided is the above modified thermostable Pol B DNA polymerase, wherein the modifier reagent is selected from the group consisting of maleic anhydride, a substituted maleic anhydride, citraconic anhydride, cis-aconitic anhydride, 2,3- dimethylmaleic anhydride, exo-cis-3,6-endoxo-δ4 - tetrahydropthalic anhydride; or 3,4,5, 6-tetrahydrophthalic anhydride.

Also provided is the above modified thermostable Pol B DNA polymerase, wherein the elevated temperature is ≥ 95°C, ≥ 96.5 °C, or ≥ 98 °C, optionally wherein the polymerase activity and the 3'-5' exonuclease activity have been restored by incubating the thermostable Pol B DNA polymerase in a reaction buffer having a pH of between 7.5 and 8.5.

Also provided is the above modified thermostable Pol B DNA polymerase, which is able to produce detectable amplification product in a polymerase chain reaction upon incubation at a temperature which is greater than 50 °C for a time sufficient to reactivate the Pol B DNA polymerase.

In certain embodiments, the modified thermostable Pol B DNA polymerase according to the present invention is obtained by using maleic anhydride or a substituted maleic anhydride as a modifier reagent. In certain embodiments, the modifier reagent is citraconic anhydride, cis-aconitic anhydride, 2,3-dimethylmaleic anhydride, exo-cis-3,6-endoxo-δ⁴ - tetrahydropthalic anhydride; or 3,4,5,6-tetrahydrophthalic anhydride.

In certain embodiments, the polymerase activity and the 3'-5' exonuclease activity have been restored by incubating the thermostable Pol B DNA polymerase in a reaction buffer having a pH of between about 7.5 and about 8.5. In certain embodiments, the polymerase activity and the 3'-5' exonuclease activity have been restored by incubating the thermostable Pol B DNA polymerase in a reaction buffer having a pH of about 8.0.

Disclosed is a method for modifying a thermostable Pol B DNA polymerase, the method comprising reacting a thermostable Pol B DNA polymerase and a modifier reagent of Formula I

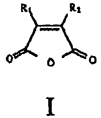

wherein R₁ and R₂ are hydrogen or a C₁-C₄ alkyl which may be linked, and wherein the reaction results in inactivation of the thermostable Pol B DNA polymerase activity and the 3'-5' exonuclease activity, and wherein the thermostable Pol B DNA polymerase activity and the 3'-5' exonuclease activity are restorable.

Accordingly, the present invention provides a method for modifying a thermostable Pol B DNA polymerase, the method comprising reacting a thermostable Pol B DNA polymerase and a modifier reagent of Formula I 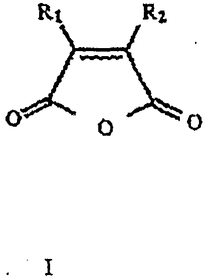 wherein R₁ and R₂ are hydrogen or a C₁-C₄ alkyl which may be linked, and wherein the reaction results in inactivation of the thermostable Pol B DNA polymerase activity and the 3'-5' exonuclease activity, wherein the thermostable Pol B DNA polymerase activity and the 3'-5' exonuclease activity are restorable by incubating the thermostable Pol B DNA polymerase at an elevated temperature that is > 50 °C, and wherein the Pol B DNA polymerase is a fusion protein which comprises Pfu, KOD, TIi, or Pfx, or a DNA polymerase sold under the trade names Vent®, Deep Vent®, Phusion™, or iProof™, or a fragment thereof having DNA polymerase activity and 3'-5' exonuclease activity and a nucleic acid binding polypeptide.

The present invention also provides a method for amplifying a target nucleic acid contained in a sample comprising the steps of:
a) contacting the sample with an amplification reaction mixture containing a primer complementary to the target nucleic acid and a modified thermostable Pol B DNA polymerase according to the invention; and
b) incubating the resulting mixture of step (a) at a temperature which is greater than about 50 °C for a time sufficient to reactivate the pol B DNA polymerase and allow formation of primer extension products.

Also provided is the above method, wherein said amplification is a polymerase chain reaction.

In certain embodiments, the present invention provides a polymerase chain reaction amplification reaction mixture, comprising at least a primer and a modified thermostable Pol B DNA polymerse of the present invention. In certain embodiments, the present invention provides a kit comprising a polymerase chain reaction amplification reaction mixture of the present inveniton and a suitable container.

These and other features of the present teachings will become more apparent from the description herein.

### BRIEF DESCRIPTION OF THE DRAWINGS

Other objects, advantages and novel features of the present invention will become apparent from the following detailed description when considered in conjunction with the accompanying drawings. The skilled artisan will understand that the drawings, described below, are for illustration purposes only.
Figure 1 shows that ECA001G had no detectable polymerase activity (denoted as Pol) and exonuclease activity (denoted as Exo) before activation, while showed both activities after activation. (See details in Example 2.1)
Figure 2 shows the effect of pH on reactivation. (See details in Example 2.2)
Figure 3 shows that AmpliTaq DNA polymerase makes more non-specific products than AmpliTaq Gold, which is less specific than ECA001Gold. (See details in Example 3)
Figure 4 shows that Pol B enzymes have lower polymerase activity than Taq at room temperature relative to their respective peak activities.
Figure 5 shows that after modificaiton and re-activation, the exonuclease to polymerase activity ratio of AmpliTaq has been changed. (See details in Example 5)

### DESCRIPTION OF VARIOUS EMBODIMENTS

DNA polymerases are known to those skilled in the art. DNA polymerases include DNA-dependent polymerases, which use DNA as a template, or RNA-dependent polymerases, such as reverse transcriptase, which use RNA as a template.

Based on sequence homology, DNA polymerases can be subdivided into seven different families: A, B, C, D, X, Y, and RT. DNA-dependent DNA polymerases fall into one of six families (A, B, C, D, X, and Y), with most falling into one of three families (A, B, and C). See, e.g., Ito et al. (1991) Nucleic Acids Res. 19:4045-4057; Braithwaite et al. (1993) Nucleic Acids Res. 21:787-802; Filee et al. (2002) J. Mol. Evol. 54:763-773; and Alba (2001) Genome Biol. 2:3002.1-3002.4. Certain DNA polymerases may be single-chain polypeptides (e.g., certain family A and B polymerases) or multi-subunit enzymes (e.g., certain family C polymerases) with one of the subunits having polymerase activity. *Id.* A fusion protein may comprise a DNA polymerase selected from a family A, B, C, D, X, or Y polymerase.

Family A polymerases ("Pol A") include both replicative and repair polymerases. Replicative members from this family include the extensively studied T7 DNA polymerase as well as the eukaryotic mitochondrial DNA Polymerase γ. Among the repair polymerases are *E. coli* DNA Pol I, *Thermus aquaticus* Pol I (Taq DNA polymerase), and *Bacillus stearothermophilus* Pol I. These repair polymerases are involved in excision repair and processing of Okazaki fragments generated during lagging strand synthesis. Because most thermostable Pol A enzymes do not possess the 3' to 5' exonuclease activity, they are incapable of proof reading the newly synthesized nucleic acid strain and consequently have high error rates. For example, Taq DNA polymerase is known to have one of the highest error rate among the commercially available polymerases.

Family B polymerases ("Pol B") mostly contain replicative polymerases and include the major eukaryotic DNA polymerases α, δ, ε, and also DNA polymerase ζ. Pol B polymerases also include DNA polymerases encoded by some bacteria and bacteriophages, of which the best characterized are from T4, Phi29 and RB69 bacteriophages. These enzymes are involved in both leading and lagging strand synthesis. A hallmark of the Pol B family of polymerases is their remarkable accuracy during replication and many have strong 3'-5' exonuclease activity (except DNA polymerase α and ζ which have no proofreading activity).

Based on their crystal structures, thermostable Pol B enzymes have a donut-like structure (see e.g. Hopfner et al., Crystal structure of a thermostable type B DNA polymerase from Thermococcus gorgonarius. Proc Natl Acad Sci U S A. (1999) 96:3600-5; Hashimoto et al., Crystal structure of DNA polymerase from hyperthermophilic archaeon Pyrococcus kodakaraensis KOD1. J Mol Biol. (2001) 306:469-77). The enzymes have a large crevice at the center as the active site for polymerase activity. Protruding from the center but on the surface of the "donut" are three clefts named as cleft D, cleft T and cleft E respectively for binding of double stranded DNA, binding of single stranded template and editing errors at the 3' end of the primer respectively (Hopfner et al., *supra*). Cleft E, where the proofreading active center resides, is substantially narrower than the central crevice where the polymerase activity center resides.

The instant inventors discovered that Cleft-E is easily accessed by small modification reagents like citraconic anhydride, while not easily accessed by bulky counter parts like diphenylmaleic anhydride. By carefully choosing the modification reagent, in various embodiments, the instant invention enables the achievement of inactivation of both of DNA polymerase activity and exonuclease activity of Pol B at a low temperature (50 °C or lower), and restoration of both enzymatic activities at an elevated temperature, while maintaining a proper ratio between the two enzyme activities. It is readily recognized that such a proper ratio is important for satisfactory PCR results. For example, if the exonuclease activity is too high compared to the polymerase activity, it would be impossible to achieve meaningful chain-elongation, at least it would be difficult to obtain sufficiently long PCR products. On the other hand, if the exonuclease activity is too low compared to the polymerase activity, inadequate proof reading and high error rate will result.

It has now been surprisingly discovered that certain dicarboxylic acid anhydrides, specifically those with sufficiently small substituent groups on the ring, are able to access cleft E of Pol B polymerases where the proofreading active center resides, and to inactivate the 3'-5' exonuclease activity of the polymerase, as well as to inactivate the 5'-3' polymerase activity of Pol B enzymes. The nuclease and polymerase activities are both recoverable after a heat reactivation step, while maintaining proper ratios of the two activities to achieve high fidelity and high efficiency DNA amplification. In other words, it has now been surprisingly discovered that heat reversable modification of Pol B enzyems by small dicarboxic acid anhydrides can eliminate primer dimer formation, yet allow high amplificaiton fidelity.

Accordingly, in various embodiments, the present invention provides methods and compositions for reversibly inactivating a thermostable Pol B DNA polymerase, a reversibly inactivated thermostable Pol B DNA polymerase, and methods and reagents for using the enzyme for performing "hot-start PCR."

### Thermostable Pol B DNA Polymerases

As discussed above, a hallmark of the B family of DNA polymerases is their remarkable accuracy during replication and many have strong 3'-5' exonuclease activity, and several thermostable Pol B DNA Polymerases are available commercially, including those described as Pfu, KOD, Tli and Pfx, or sold under the trade names Vent@, Deep Vent@, Phusion™, iProof™, as well as the fusion proteins disclosed in U.S. Pat. App. No. 20060228726. Suitable thermostable Pol B DNA polymerases include naturally occurring Pol B polymerases as well as chimeric or recombinant Pol B DNA polymerases.

The Pol B DNA polymerase is a modified thermostable Pol B DNA polymerase having both a DNA polymerase activity and a 3'-5' exonuclease activity, wherein the modified thermostable Pol B DNA polymerase is produced by reacting, under essentially aqueous conditions, a thermostable Pol B DNA polymerase with a modifier reagent of Formula I 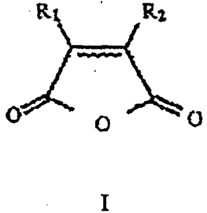 wherein R₁ and R₂ are hydrogen or a C₁-C₄ alkyl which may be linked, wherein the reaction results in inactivation of the thermostable Pol B DNA polymerase activity and the 3'-5' exonuclease activity,
wherein a ratio between the polymerase activity and the 3'-5' exonuclease activity is restorable to a value that is essentially the same as before modification,
wherein the thermostable Pol B DNA polymerase activity and the 3'-5' exonuclease activity are restorable by incubating the thermostable Pol B DNA polymerase at an elevated temperature that is > 50 °C,
and wherein the Pol B DNA polymerase is a fusion protein which comprises Pfu, KOD, TIi, or Pfx, or a DNA polymerase sold under the trade names Vent®, Deep Vent®, Phusion™, or iProof™, or a fragment thereof having DNA polymerase activity and 3'-5' exonuclease activity and a nucleic acid binding polypeptide.

The family B polymerase can be an archaeal family B polymerase, such as a polymerase from the genus Thermococcus, Pyrococcus, or Pyrobaculum. The family B polymerase can be Pfu DNA polymerase or a fragment or variant thereof having polymerase activity.

### Certain Chemical Modifiers

In various embodiments, the present invention relates to methods for modifying thermostable Pol B DNA polymerases whereby the 3'-5' exonuclease and DNA polymerase activities are completely or nearly completely inactivated, and wherein the inactivation can be reversed at an elevated temperature under suitable conditions.

According to certain embodiments of the present invention, thermostable Pol B DNA polymerases is reversibly inactivated by reacting with a suitable chemical compound or chemical modifier. Preferably the chemical modifier may be a dicarboxylic acid anhydride. As indicated above, reversible inactivation of DNA polymerase activities was known in the art to reversibly inactivate the enzymes by blocking the ε-amino group of the lysine of the active site. However, it has been heretofore unknown whether the 5'-3' polymerase and 3'-5' exonuclease active sites are able to be reversibly inactivated by dicarboxylic acid anhydride molecules, such that the resultant re-activated polymerase will function in PCR.

In some embodiments, chemical modifiers for the present invention include dicarboxylic acid anhydrides of formula I: 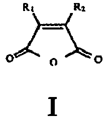 wherein R₁ and R₂ are hydrogen or a C₁-C₄ alkyl which may be linked. R₁ and R₂ may be directly attached to the ring by a carbon-carbon bond or through a carbon-heteroatom bond such as a carbon-oxygen, carbon-nitrogen, or carbon-sulfur bond. The organic radicals may also be linked to each other to form a ring structure as in, for example, 3,4,5,6-tetrahydrophthalic anhydride.

Dicarboxylic acid anhydrides are known to react with the amino groups of proteins to give corresponding acylated products (see e.g. U.S. Pat. No 5,677,152). *See also* Palacian et al., 1990, Mol. Cell. Biochem. 97:101-111, incorporated herein by reference, for descriptions of plausible mechanisms for both the acylation and deacylation reactions. The *cis*-carbon-carbon double bond, and potentially other substituents, are believed to enhance the reversibility of the above dicarboxylic acid anhydrides, because they maintain the terminal carboxyl group of the acylated residues in a spatial orientation suitable for interaction with the amide group, and subsequent deacylation.

Examples of suitable chemical modifying reagents suitable for the present invention include maleic anhydride; substituted maleic anhydrides such as citraconic anhydride, cis-aconitic anhydride, and 2,3-dimethylmaleic anhydride; exo-cis-3,6-endoxo-δ⁴ -tetrahydropthalic anhydride; and 3,4,5,6-tetrahydrophthalic anhydride. The reagents are commercially available from, for example, Aldrich Chemical Co. (Milwaukee, Wis.), Sigma Chemical Co. (St. Louis, Mo.), or Spectrum Chemical Mfg. Corp. (Gardena, Calif.). Modifications of Pol B thermostable DNA polymerases using the substituted maleic anhydride reagents citraconic anhydride and cis-aconitic anhydride are described in the Examples.

### Preparation of the Reversibly Inactivated Thermostable Enzymes

In some embodiments, modification of Pol B DNA polymerase includes reacting molecules of a Pol B thermostable DNA polymerase enzyme, with a chemical modifier at a suitable temperature, for example at 4 °C, at generally an alkaline pH, under aqueous conditions, for sufficient time to reduce the activity of the enzyme to acceptable levels.

The modification typically can be carried out within 1 to 12 hours reaction time, or for less than 60 minutes, most preferably 15 to 30 minutes, but this will vary slightly depending on the reagent used.

Suitable reaction conditions are known in the art and are described further herein and in the examples. It is known that chemical modification of lysine residues is favored at alkaline pH. The modification reaction is carried out at pH 7-10, preferably at between 7.0 and 9.0, or more preferably at between 7-8.5, in an aqueous buffer at a temperature at or below room temperature. The rate of hydrolysis increases with pH, but hydrolysis which occurs at pH greater than about 9 can result in suboptimal acylation of the protein.

The reaction is essentially complete following an incubation for about one hour, but may last as long as 12-24 hours.

Dicarboxylic acid anhydrides react easily with water to give the corresponding acids. Therefore, a large fraction of the reagent is hydrolyzed during modification of the protein amino groups.

In general, a molar excess of the modifier reagent relative to the protein is used in the acylation reaction, and the optimal molar ratio is determined empirically. As an example, ECA001 is essentially completely inactivated (less than 5%, preferably less than 1%, more preferably less than 0.1% of the original activity) by a reaction with a 20-fold or greater molar excess of citraconic anhydride.

In the methods of the present invention, both the polymerase and 3'-5' exonuclease activities of the thermostable Pol B DNA polymerase are completely inactivated (less than 1% of their original activity) or nearly completely inactivated (less than 5% of their original activity). The modified thermostable enzymes of the present invention do not have any significant detectable activity when assayed at 37 °C, even after incubation at 37 °C for an hour or more. On the other hand, enzymatic activity of the present chemically modified enzymes is increased three times within thirty minutes when incubated at a more elevated temperature, i.e., above 50 °C., preferably at a temperature of 75 °C to 100 °C, and most preferably at 98 °C. Such chemically modified enzymes may be employed in all applications involving a thermostable Pol B DNA polymerase, such as DNA amplification, ligation, exonucleolytic or endonucleolytic reactions.

An important aspect of the heat-activatable enzymes of the present invention is their storage stability. In general, the enzymes described herein are stable for extended periods of time, which eliminates the need for preparation immediately prior to each use. For example, citraconylated ECA001 was found to remain inactivated for at least four weeks when stored at 25 °C.

### Amplification Methods

The thermostable Pol B DNA polymerases of the present invention can be used for primer-based nucleic acid synthesis or amplification reactions. The methods of the present invention involve the use of a reaction mixture containing a reversibly inactivated thermostable Pol B DNA polymerase and subjecting the reaction mixture to a high temperature incubation prior to, or as an integral part of, the amplification reaction. The high temperature incubation results in deacylation of the amino groups and recovery of both the polymerase and 3'-5' exonuclease activities.

The deacylation of the modified amino groups, and the reactivation of the enzyme activities, result from both the increase in temperature and a concomitant decrease in pH. Amplification reactions typically are carried out in a Tris-HCl buffer formulated to a pH of 8.0 to 9.0 at room temperature. At room temperature, the alkaline reaction buffer conditions favor the acylated form of the amino group. Although the pH of the reaction buffer is adjusted to a pH of 8.0 to 9.0 at room temperature, the pH of a Tris-HCl reaction buffer decreases with increasing temperature. Thus, the pH of the reaction buffer is decreased at the elevated temperatures at which the amplification is carried out and, in particular, at which the activating incubation is carried out. The decrease in pH of the reaction buffer favors deacylation of the amino groups.

It is well recognized that buffer system used in a reaction determines the pH changes that occur as a result of high temperature reaction conditions, and the temperature dependence of pH for various buffers used in biological reactions is well-known (see e.g. Good et al., 1966, Biochemistry 5(2):467-477). Although amplification reactions are typically carried out in a Tris-HCl buffer, amplification reactions may be carried out in buffers which exhibit a smaller or greater change of pH with temperature. Depending on the buffer used, a more or less stable modified enzyme may be desirable. For example, using a modifying reagent which results in a less stable modified enzyme calls for recovery of sufficient enzyme activity under smaller changes of buffer pH.

Activation of the modified enzyme is preferably achieved by an incubation at a temperature which is equal to or higher than the primer hybridization (annealing) temperature used in the amplification reaction to insure primer binding specificity. The length of incubation required to recover enzyme activity depends on the temperature and pH of the reaction mixture and on the stability of the acylated amino groups of the enzyme, which depends on the modifier reagent used in the preparation of the modified enzyme. A wide range of incubation conditions are usable; optimal conditions may be determined empirically for each reaction. In general, an incubation is carried out in the amplification reaction buffer at a temperature greater than about 50 °C. for between about 1 seconds and about 20 minutes. Optimization of incubation conditions for the reactivation of enzymes not exemplified, or for reaction mixtures not exemplified, can be determined by routine experimentation following the guidance provided herein.

In a preferred embodiment, PCR amplification is carried out using a reversibly inactivated thermostable DNA polymerase. The annealing temperature used in a PCR amplification typically is about 50-75 °C., and the pre-reaction incubation is carried out at a temperature equal to or higher than the annealing temperature, preferably a temperature greater than about 90 °C. The amplification reaction mixture preferably is incubated at about 90-100 °C. for up to about 12 minutes to reactivate the DNA polymerase prior to the temperature cycling. Suitable pre-reaction incubation conditions for typical PCR amplifications are described in the Examples, along with the effect on amplification of varying the pre-reaction incubation conditions.

The first step in a typical PCR amplification includes heat denaturation of the double-stranded target nucleic acid. The exact conditions required for denaturation of the sample nucleic acid depends on the length and composition of the sample nucleic acid. Typically, an incubation at 90-100 °C for about 10 seconds up to about 4 minutes is effective to fully denature the sample nucleic acid. The initial denaturation step can serve as the pre-reaction incubation to reactivate the DNA polymerase. However, depending on the length and temperature of the initial denaturation step, and on the modifier used to inactivate the DNA polymerase, recovery of the DNA polymerase activity may be incomplete. If maximal recovery of enzyme activity is desired, the pre-reaction incubation may be extended or, alternatively, the number of amplification cycles can be increased.

In certain embodiments, the modified enzyme and initial denaturation conditions are chosen such that only a fraction of the recoverable enzyme activity is recovered during the initial incubation step. Subsequent cycles of a PCR, which each involve a high-temperature denaturation step, result in further recovery of the enzyme activity. Thus, activation of enzyme activity is delayed until after the initial cycling of the amplification. This "time release" of DNA polymerase activity has been observed to further decrease non-specific amplification. It is known that an excess of DNA polymerase contributes to non-specific amplification. In the present methods, the amount of DNA polymerase activity present is low during the initial stages of the amplification when the number of target sequences is low, which reduces the amount of non-specific extension products formed. Maximal DNA polymerase activity is present during the later stages of the amplification when the number of target sequences is high, and which enables high amplification yields. If necessary, the number of amplification cycles can be increased to compensate for the lower amount of DNA polymerase activity present in the initial cycles.

An advantage of the methods of the present invention is that the methods require no manipulation of the reaction mixture following the initial preparation of the reaction mixture. Thus, the methods are ideal for use in automated amplification systems and with in-situ amplification methods, wherein the addition of reagents after the initial denaturation step or the use of wax barriers is inconvenient or impractical.

The methods of the present invention are particularly suitable for the reduction of non-specific amplification in a PCR with high polymerization fidelity. Accordingly, the compositions and methods of the present invention are particularly suitable for amplification of target nucleic acid sequences and for cloning of such long sequences, such as in genomics studies.

The methods and compositions of the present invention, however, are not restricted to any particular amplification system. The reversibly-inactivated enzymes of the present invention can be used in any primer-based amplification system which uses thermostable Pol B DNA polymerases and relies on reaction temperature to achieve amplification specificity. The present methods can be applied to isothermal amplification systems which use thermostable enzymes. Only a transient incubation at an elevated temperature is required to recover enzyme activity. After the reaction mixture is subjected to a high temperature incubation in order to recover enzyme activity, the reaction is carried out at an appropriate reaction temperature. Other amplification methods in addition to PCR (U.S. Pat. Nos. 4,683,195; 4,683,202; and 4,965,188) include, but are not limited to, the following: Ligase Chain Reaction (LCR, Wu and Wallace, 1989, Genomics 4:560-569 and Barany, 1991, Proc. Natl. Acad. Sci. USA 88:189-193); Polymerase Ligase Chain Reaction (Barany, 1991, PCR Methods and Applic. 1:5-16); Gap-LCR (PCT Patent Publication No. WO 90/01069); Repair Chain Reaction (European Patent Publication No. 439,182 A2), 3SR (Kwoh et al 1989, Proc. Natl. Acad. Sci. USA 86:1173-1177; Guatelli et al 1990, Proc. Natl. Acad. Sci. USA 87:1874-1878; PCT Patent Publication No. WO 92/0880A), and NASBA (U.S. Pat. No. 5,130,238). A recent survey of amplification systems was published in Abramson and Myers, 1993, Current Opinion in Biotechnology 4:41-47.

Sample preparation methods suitable for each amplification reaction are described in the art (see, for example, Sambrook et al., *supra,* and the references describing the amplification methods cited above). Simple and rapid methods of preparing samples for the PCR amplification of target sequences are described in Higuchi, 1989, in PCR Technology (Erlich ed., Stockton Press, New York), and in PCR protocols, Chapters 18-20 (Innis et al., ed., Academic Press, 1990). One of skill in the art will be able to select and empirically optimize a suitable protocol.

Methods for the detection of amplified products have been described extensively in the literature. Standard methods include analysis by gel electrophoresis or by hybridization with oligonucleotide probes. The detection of hybrids formed between probes and amplified nucleic acid can be carried out in variety of formats, including the dot-blot assay format and the reverse dot-blot assay format, including Saiki et al, 1986, Nature 324:163-166; Saiki et al., 1989, Proc. Natl. Acad. Sci. USA 86:6230; PCT Patent Publication No. 89/11548; U.S. Pat. Nos. 5,008,182, and 5,176,775; PCR Protocols: A Guide to Methods and Applications (ed. Innis et al., Academic Press, San Diego, Calif.) :337-347. Reverse dot-blot methods using microwell plates are described in e.g. U.S. Pat. No. 5,232,829; Loeffelholz et al., 1992, J. Clin. Microbiol. 30(11):2847-2851; Mulder et al., 1994, J. Clin. Microbiol. 32(2):292-300; and Jackson et al., 1991, AIDS 5:1463-1467.

Another suitable assay method, referred to as a 5'-nuclease assay, is described in U.S. Pat. No. 5,210,015; and Holland et al, 1991, Proc. Natl. Acad. Sci. USA 88:7276-7280. In the 5'-nuclease assay, labeled probes are degraded concomitant with primer extension by the 5' to 3' exonuclease activity of the DNA polymerase, e.g., Taq DNA polymerase. Detection of probe breakdown product indicates both that hybridization between probe and target DNA occurred and that the amplification reaction occurred.

An alternative method for detecting the amplification of nucleic acid by monitoring the increase in the total amount of double-stranded DNA in the reaction mixture is described in Higuchi et al., 1992, Bio/Technology 10:413-417; Higuchi et al., 1993, Bio/Technology 11:1026-1030; and European Patent Publication Nos. 487,218 and 512,334. The detection of double-stranded target DNA relies on the increased fluorescence that ethidium bromide (EtBr) and other DNA binding labels exhibit when bound to double-stranded DNA. The increase of double-stranded DNA resulting from the synthesis of target sequences results in a detectable increase in fluorescence. A problem in this method is that the synthesis of non-target sequence, i.e., non-specific amplification, results in an increase in fluorescence which interferes with the measurement of the increase in fluorescence resulting from the synthesis of target sequences. Thus, the methods of the present invention are particularly useful because they reduce non-specific amplification, thereby minimizing the increase in fluorescence resulting from the amplification of non-target sequences.

In addition to using the modified, inactivated nucleic acid polymerases as described above, PCR reactions may be improved by using additives that affect the meting behavior of nucleic acids in the reaction mixture. For example, difficult PCR amplifications, such as reactions that yield non-specific products, and especially amplification of templates having a high GC content or having extensive secondary structure, may be improved by employing additives that "isostabilize" AT- and GC-base pairing to the level of AT-base pair stability. Suitable such PCR additives include multifunctional polyols, preferably trifunctional polyols, most preferably glycerol; amides, preferably carbamides, most preferably formamide; alkaline ammonia salts, preferably alkylated ammonia salts, most preferably tetramethylammonium chloride; sulfoxides, preferably alkylated sulfoxides, most preferably dimethylsulfoxide; sulfates, preferably inorganic sulfates, most preferably ammonium sulfate polyalkylene glycols, most preferably polyethylene glycol. Additionally, SSB protein (single strand binding protein), preferably E. coli SSB protein (see, Schwarz et al., E. coli SSB protein, Nucleic Acids Research, 18: 1079 (1990)), T4 gene 32 protein, or yeast SSB protein, may also be used. Preferred PCR additives also include calf thymus protein UP1 (see, Amrute et al., Biochemistry, 33(27): 8282-8291 (1994)).

A particularly preferred PCR additive for this purpose is betaine (1-carboxy-N,N,N-trimethyl-methanaminium inner salt) and other zwitterionic bases characterized by the -OOC-CH₂-NMe₃⁺ group (collectively "betaines"). The PCR additives are advantageously added to a PCR reaction mixture in an amount effective to improve the specificity of the amplified product. Typically concentrations of additive from 1 mM to 5M, preferably about 1M, are used, however any amount that improves the yield of the specific amplification product, compared with a PCR reaction carried out in the absence of the additive, is suitable.

In PCR reactions involving reverse transcription (RT-PCR), the materials and methods of the present invention permit a continuous reaction to be carried out in one vessel, without interrupting the enzymatic reactions by additional handling steps.

The present invention also relates to kits, multicontainer units comprising useful components for practicing the present method. A useful kit contains a reversibly-inactivated thermostable Pol B DNA polymerase and one or more reagents for carrying out an amplification reaction, such as oligonucleotide primers, substrate nucleoside triphosphates, cofactors, and an appropriate buffer.

In certain embodiments, the present invention relates to kits comprising such an inactivated thermostable enzyme together with a Tris-buffered reaction buffer or Tris-buffered reaction mixture.

Disclosed is the use of RNase H positive and RNase H negative reverse transcriptases in combination with inactivated pol B DNA polymerase for a continuous reverse transcription polymerase chain reaction (RT-PCR) to be performed in a single reaction tube without interrupting the enzymatic reactions by additional handling steps.

The examples of the present invention presented below are provided only for illustrative purposes and not to limit the scope of the invention.

Standard protocols of molecular biology applications, enzymology, protein and nucleic acid chemistry are well described in printed publications such as Molecular Cloning-A Laboratory Manual, Cold Spring Harbor, N.Y. (Sambrook et al. 1989); PCR Protocols-A Guide to Methods and Applications, Academic Press, N.Y. (Innis et al., eds, 1990), PCR Primer-A Laboratory Manual, CSHL Press (Dieffenbach and Dveksler, eds., 1995); and Methods in Enzymology, Academic Press, Inc.

Aspects of the present teachings may be further understood in light of the following examples, which should not be construed as limiting the scope of the present teachings in any way.

### EXAMPLES

### Example 1 Modification of ECA001

A Family B DNA polymerase fusion protein, designated ECA001 was used for modification according to a method of the present invention. ECA001 comprises a nucleic acid binding polypeptide Pae3192 from the crenarchaeon *Pyrobaculum aerophilum,* joined to the C-terminus of full length Pfu DNA polymerase. ECA001 was described as 10His-Pfu-Pae3192 in U.S. Pat. App. No. 20060228726, which is incorporated herein by reference in its entirety. ECA001 was constructed as follows. An NdeI-XhoI restriction fragment comprising a polynucleotide sequence encoding full length Pfu DNA polymerase in frame with a polynucleotide sequence encoding Pae3192 was cloned into the NdeI and XhoI sites of the pET16b vector (Novagen, Milwaukee, Wis.) using standard recombinant methods. The resulting recombinant vector (pDS2r) encodes a fusion protein comprising Pae3192 joined to the C-terminus of Pfu DNA polymerase by a Gly-Thr-Gly-Gly-Gly-Gly peptide linker. A 10xHis affinity tag is present at the N-terminus of the fusion protein.

Modification of ECA001 was carried out as follows: 50 ml of 27 mM citraconic anhydride (CAD) in DMF was mixed with 1 liter of purified ECA001 that had been kept at 4 °C and adjusted to A₂₈₀= 1 in absorbance and 9.0 in pH with buffer containing 50 mM Tris, pH9, 0.1mM EDTA, and 0.01% Tween. The mixture was kept mixing for 60 minutes at 4 °C before transferring to a storage buffer. The CAD to enzyme ratio describe above is about 100 to 1, and other ratios, such as 800:1, 400:1, 200:1, 100:1, 50:1 and 20:1 can be used by adjusting the concentration of CAD that is to be mixed with enzyme solution.

The resulting modified protein is referred to as ECA001Gold or ECA001G.

### Example 2 Modified ECA001G Has No Detectable DNA polymerase and 3'-5' nuclease activities

This example demonstrates that (1) after modification, no DNA polymerase and 3'-5' nuclease activities were detectable before activation, and become detectable after heat activation; (2) ECA001G is optimally activated under pH of 8, and activated enzyme is useful in PCR; (3) ECA001G improves PCR by eliminating the formation of primer-dimers.

**Example 2.1** This example is intended to demonstrate that ECA001G is inactive in both polymerase and exonuclease activity before activation, while such activities are restored after heat-activation. To this end, two activity assays, polymerase activity assay and exonuclease activity assay, were used to test protein samples (1) before modification, (2) after modification but before re-activation, and (3) after modification and re-activation, respectively.

Activity assays contain 1x AmpliTaq Gold buffer, 1.5 mM MgC12, 50 mM KC1, 0.25 mM each of dNTP and 500 nM of substrate. For polymerase activity assay, a substrate referred to as Polsub14 that has the sequence from 5' to 3': 
BHQ1-ATCATCATATCATCAACTGGCCGTCGTTTACATATGTAAAACGAC-
GGCCAG(FAM-dT)T (SEQ ID NO:1)
 is used. Where, BHQ1 is Blackhole Quencher 1, a product from Biosearch Biotechnologies, Novato, Calif., and FAM-dT is deoxythymine with fluorescein (FAM) linked to the base. FAM is a fluorophore which emits at 520 nm and can be quenched by BHQ1® when they stay in close proximity. The structure is shown as below:

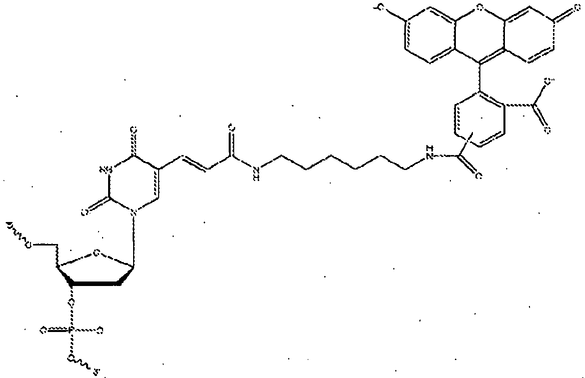

Polsub14 assumes a hairpin structure in its native state, at which the polymerase activity is assayed as shown below:

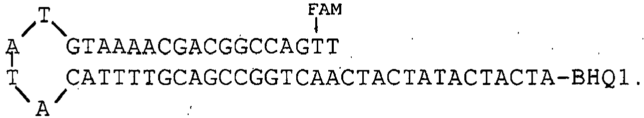

The fluorescein (FAM) FRET donor attached to the base of dT at the penultimate position is separated from BHQ1 mostly by a single stranded region. This conformation allows BHQ1 to quench fluorescein effectively. Once the substrate is polymerized, the single stranded region becomes double stranded as shown below: 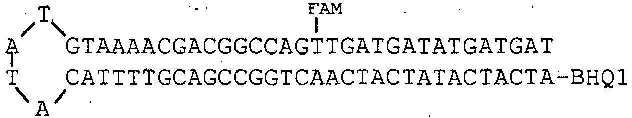 which separates BHQ1 from the fluorescein by a stiffer double stranded DNA, rendering the fluorescein to be de-quenched.

For the 3'-5' nuclease assay, the substrate (Exo-MM HP) has the sequence
BHQ-TTTTCTGGCCGTCGTTTTACATATGTAAAACGACGGCCAG(FAM-dT) (SEQ ID NO:2), which also forms a hairpin structure in its native state shown below.

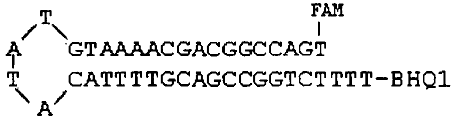

This substrate has two features: (1) The FAM attached to the base of dT at 3'-end is quenched by BHQ1; (2) The FAM-attaching dT mismatches with its template. As Pol B enzymes binds to this hairpin, 3'-5' nuclease activity will recognize the mismatch and excise the base together with the FAM attached thereto, resulting in the de-quenching of FAM.

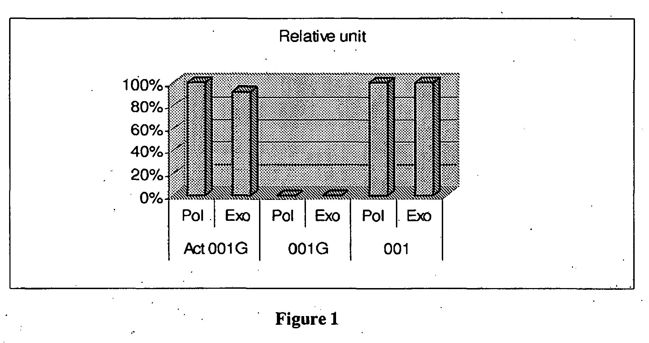

Activation was carried out at 98 °C for 10 minutes in the reaction buffer, without substrate. Figure 1 shows the activities determined at 60 °C, before and after activation for 1.5 units of enzyme in 100 µl.

As shown in Figure 1, ECA001G (designated as 001G) showed no detectable polymerase activity before activation, while showed activity after activation (designated as Act 001G). Unmodified enzyme, ECA001G, (designated as 001) was used as a control.

**Example 2.2.** This example serves to demonstrate that ECA001G is optimally activated at pH 8, and the activated enzyme is useful in PCR.

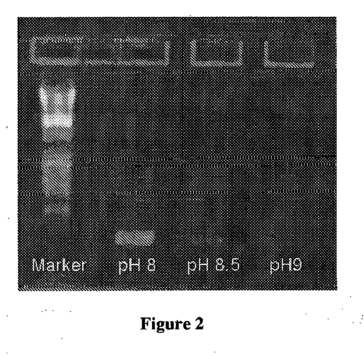

PCR reactions were performed with 5 unit/100 µl of ECA001G, in 50 mM Tris, at pH 8, 8.5 and 9 respectively with 1.5 mM MgCl₂, 50 mM KCl, 0.25 mM each of dNTP, 10⁵ copies of a plasmid containing a full length of GAPDH cDNA clone, and 500 nM each of GAPDH forward (GAAGGTGAAGGTCGGAGTC) (SEQ ID NO:3) and GAPDH reverse (GAAGATGGTGATGGGATTTC) (SEQ ID NO:4) primers. The cycling condition was 10 minutes activation at 98 °C followed by 40 cycles of 15 seconds at 98 °C and 1 minutes at 60 °C. After the cycling was finished, the PCR products were analyzed via agarose gel electrophoresis. The result is shown in Figure 2. Activation at pH 8 gave the highest yield; while pH 8.5 gave suboptimal yield; and pH 9 gave no amplification product.

### Example 3. ECA001G Successfully Amplified Target Nucleic Acids Under Demanding PCR Conditions

This experiment demonstrates the usefulness of ECA001G under demanding PCR conditions. Under these conditions, if hot-start techniques were not employed, primer dimers tend to form in the PCR reaction mix. With hot-start properties PCR reactions are devoid of primer dimers or showed diminished interference by primer-dimers.

This example includes reactions which is referred to as PCR Residual Activity Assay that involved three set of primers respectively The primer sets are commercially available from Applied Biosystems, Foster City, Calif. Specifically, Set 1 is Hs00378363_g1; Set 2, Hs00399572_m1 and Set 3, Hs00372859_m1.

The components are listed below:
1 x PCR Buffer
1X MgCl₂
0.2 mM each dNTP
50 Unit/ml DNA polymerase
1 x primer mixes
and 0.8 ng/µl cDNA (human)
Due to the needs of the individual polymerases, 1X buffer and 1X MgCl₂ are different as stated below:

| | AmpliTaq | AmpliTaq Gold | ECA001 | ECA001 Gold |
|---|---|---|---|---|
| Buffer | 1X PCR Buffer II (ABI) | 1X AmpliTaq Gold buffer | 10 mM Tris, pH 8.9, 90 mM KCl | 1X AmpliTaq Gold buffer with 40 mM extra KCl |
| MgCl₂ | 4.5 mM | 4.5 mM | 1.5 mM | 1.5 mM |

The cycling conditions for ECA001 are listed below:
Preincubation 25 °C for 60 min.
98 °C for 30 sec
40 cycles of:
   98 °C for 5 sec
   60 °C for 1 min

The cycling conditions for ECA001-Gold
Preincubation 25 °C for 60 min
95 °C for 10 min
40 cycles of:
   98 °C for 5 sec
   60 °C for 1 min

The cycling conditions for AmpliTaq :
Preincubation 25 °C for 60 min.
95 °C for 30 sec
40 cycles of:
   95 °C for 10 sec
   60 °C for 1 min

The cycling conditions for AmpliTaq Gold:
Preincubation 25 °C for 60 min
95 °C for 10 min
40 cycles of:
   95 °C for 10 sec
   60 °C for 1 min

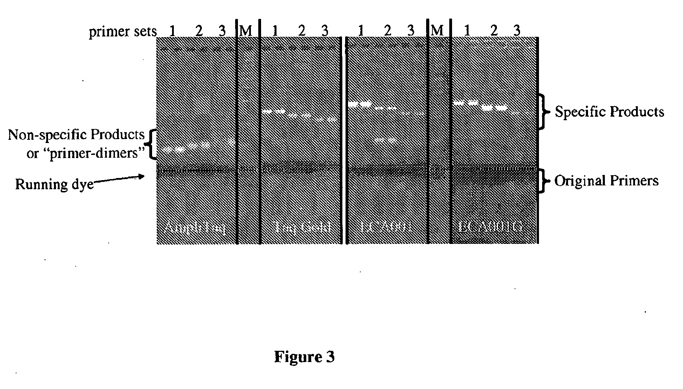

Each reaction was run in duplication. After cycling, the products were run on an ethidium bromide agarose gel as shown in Figure 3.

Figure 3 shows that AmpliTaq produced mostly non-specific/primer-dimer products for all the three primer sets under the described conditions, while AmpliTaq Gold produces mostly the specific products. ECA001 inherently formed fewer non-specific products or primer-dimers than AmpliTaq and ECA001G showed no production of non-specific/primer-dimers. These ECA001G results are comparable to the AmpliTaq Gold results in terms of the lack of non-specific products, however the yields from ECA001G were greater than those for Taq Gold, especially for primer set 2.

### Example 4 Pol B Enzymes Have Lower Polymerase Activity Than Taq At Room Temperature

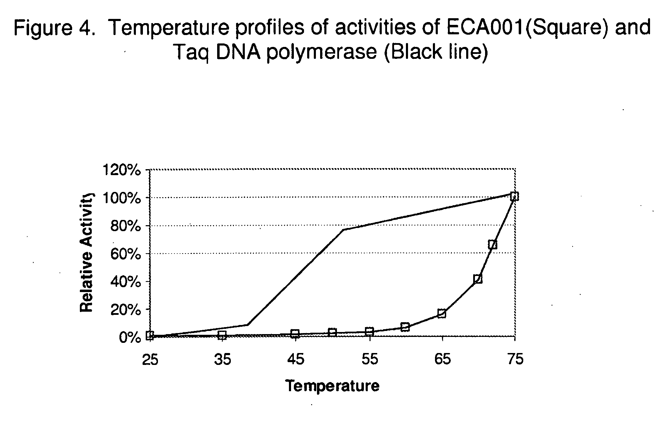

Pol B DNA polymerases enzymes have relative low polymerase activity at room temperature, while Taq DNA polymerase still retains >5% of its peak activity (Figure 4).

### Example 5: The Exonuclease to Polymerase Activity Ratio Are Unchanged After Activation

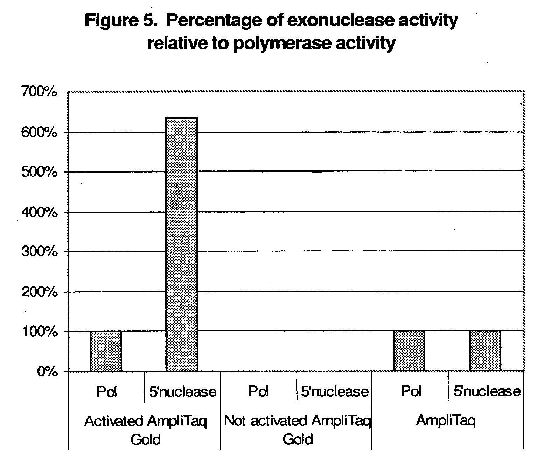

Our data (Figure 3) showed that fewer tendencies to form primer-dimer or non-specific products. It is true that lysine is a common amino acid that can be found in many enzymes. The chemicals in Birch'152 patent were proved to be able to modify 5'-3' exonuclease activity (different from proofreading activity) of Taq in our hands. However, the exonuclease to polymerase activity ratio has been changed (Figure 5).

There are several advantages to use Pol B enzymes instead of the prevailing enzyme, Taq. (1) Pol B enzymes have proof-reading activity, therefore, the error rates in PCR products are typically significantly smaller. (2) Pol B enzymes, like ECA001 tend to form less primer-dimer and non-specific products as shown in Figure 3. (3) Pol B enzymes tend not to add an extra adenosine to the final PCR product, so the product will be the exact the length defined by the two primers. In contrast, Taq will generate a mixture of +1 product and the exact product. A long post-PCR incubation at 72 °C is needed if homogenous products are desired.

### Example 6 Preparation of Inactivated KOD DNA Polymerase

Modification of KOD is carried out as follows: 50 ml of 27 mM citraconic anhydride (CAD) in DMF is mixed with 1 liter of purified KOD that have been kept at 4 °C and adjusted to A₂₈₀= 1 in absorbance and 9.0 in pH with buffer containing 50 mM Tris, pH9, 0.1mM EDTA, and 0.01% Tween. The mixture is kept mixing for 60 minutes at 4 °C before transferring to a storage buffer. The CAD to enzyme ratio describe above is about 100 to 1, and other ratios, such as 800:1, 400:1, 200:1, 100:1, 50:1 and 20:1 are also tried by adjusting the concentration of CAD mixed with the enzyme solution. Exonuclease and polymerase activities are assayed as described above for ECA001.

### Example 7 : Preparing Inactivated Family B DNA Polymerase Using 2, 3-Diphenylmaleic Anhydride

Modification of ECA001 with 2,3-diphenylmaleic anhydride was carried out as follows: 50 ml of 27 mM with 2,3-diphenylmaleic anhydride (DPMA) in DMF was mixed with 1 liter of purified ECA001 that had been kept at 4 °C and adjusted to A₂₈₀= 1 in absorbance and 9.0 in pH with buffer containing 50 mM Tris, pH9, 0.1mM EDTA, and 0.01% Tween. The mixture was mixed for 60 minutes at 4 °C before transferring to a storage buffer. The DPMA to enzyme ratio describe above was about 100 to 1, and other ratios, such as 800:1, 400:1, 200:1, 100:1, 50:1 and 20:1 were also tried by adjusting the concentration of DPMA to be mixed with enzyme solution. Exonuclease and polymerase activities were assayed as described above in Example 2.

We found that Modification of ECA001 with DPMA lead to the inactivation of the enzyme. Heating at 95 degree C for 10 minutes restored both the polymerase and 3'-5' exonuclease activities. However, the restored enzyme was not able to function in PCR reactions.

## Claims

1. A modified thermostable Pol B DNA polymerase having both a DNA polymerase activity and a 3'-5' exonuclease activity, wherein the modified thermostable Pol B DNA polymerase is produced by reacting, under essentially aqueous conditions, a thermostable Pol B DNA polymerase with a modifier reagent of Formula I 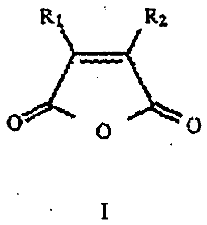 wherein R₁ and R₂ are hydrogen or a C₁-C₄ alkyl which may be linked, wherein the reaction results in inactivation of the thermostable Pol B DNA polymerase activity and the 3'-5' exonuclease activity,
wherein a ratio between the polymerase activity and the 3'-5' exonuclease activity is restorable to a value that is essentially the same as before modification,
wherein the thermostable Pol B DNA polymerase activity and the 3'-5' exonuclease activity are restorable by incubating the thermostable Pol B DNA polymerase at an elevated temperature that is > 50 °C,
and wherein the Pol B DNA polymerase is a fusion protein which comprises Pfu, KOD, TIi, or Pfx, or a DNA polymerase sold under the trade names Vent@, Deep Vent@, Phusion™, or iProof™, or a fragment thereof having DNA polymerase activity and 3'-5' exonuclease activity and a nucleic acid binding polypeptide.

2. The modified thermostable Pol B DNA polymerase of Claim 1, wherein the Pol B DNA polymerase is 10His-Pfu-Pae3192,

3. The modified thermostable Pol B DNA polymerase according to Claim 1, wherein the modifier reagent is selected from the group consisting of maleic anhydride, a substituted maleic anhydride, citraconic anhydride, cis-aconitic anhydride, 2,3- dimethylmaleic anhydride, exo-cis-3,6-endoxo-δ4 -tetrahydropthalic anhydride; or 3,4,5, 6-tetrahydrophthalic anhydride.

4. The modified thermostable Pol B DNA polymerase of Claim 1, wherein the elevated temperature is ≥ 95 °C, ≥ 96.5 °C, or ≥ 98 °C.

5. The modified thermostable Pol B DNA polymerase of Claim 4, wherein the polymerase activity and the 3'-5' exonuclease activity have been restored by incubating the thermostable Pol B DNA polymerase in a reaction buffer having a pH of between 7.5 and 8.5.

6. The modified thermostable Pol B DNA polymerase according to Claim 1, which is able to produce detectable amplification product in a polymerase chain reaction upon incubation at a temperature which is greater than 50 °C for a time sufficient to reactivate the Pol B DNA polymerase.

7. A polymerase chain reaction amplification reaction mixture, comprising at least a primer and a modified thermostable Pol B DNA polymerase of Claim 1.

8. A kit comprising a modified thermostable Pol B DNA polymerase of Claim 1, and a suitable container.

9. A method for modifying a thermostable Pol B DNA polymerase, the method comprising reacting a thermostable Pol B DNA polymerase and a modifier reagent of Formula I 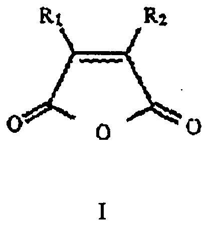 wherein R₁ and R₂ are hydrogen or a C₁-C₄ alkyl which may be linked, and wherein the reaction results in inactivation of the thermostable Pol B DNA polymerase activity and the 3'-5' exonuclease activity, wherein the thermostable Pol B DNA polymerase activity and the 3'-5' exonuclease activity are restorable as recited in claim 1, and wherein the Pol B DNA polymerase is a Pol B DNA polymerase recited in claim 1.

10. A method for amplifying a target nucleic acid contained in a sample comprising the steps of:
a) contacting the sample with an amplification reaction mixture containing a primer complementary to the target nucleic acid and a modified thermostable Pol B DNA polymerase according to Claim 1; and
b) incubating the resulting mixture of step (a) at a temperature which is greater than about 50 °C for a time sufficient to reactivate the pol B DNA polymerase and allow formation of primer extension products.

11. The method of Claim 10, wherein said amplification is a polymerase chain reaction.

## Patentansprüche

1. Eine modifizierte thermostabile Pol B DNA-Polymerase, die beides, eine DNA-Polymeraseaktivität und eine 3'-5'-Exonukleaseaktivität hat, wobei die modifizierte thermostabile Pol B DNA-Polymerase durch Reaktion, unter im Wesentlichen wässrigen Bedingungen, einer thermostabilen DNA-Polymerase mit einem modifizierenden Reagenz der Formel 1 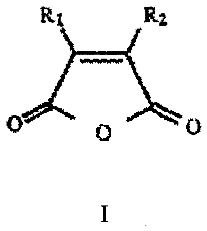 erzeugt wird,
wobei R₁ und R₂ Wasserstoff oder ein C₁-C₄ Alkyl, die verknüpft sein können, sind, wobei die Reaktion in der Inaktivierung der thermostabilen Pol B DNA-Polymeraseaktivität und der 3'-5'-Exonukleaseaktivität resultiert,
wobei ein Verhältnis zwischen der Polymeraseaktivität und der 3'-5'-Exonukleaseaktivität zu einem Betrag wiederherstellbar ist, der im Wesentlichen derselbe ist als vor der Modifikation,
wobei die thermostabile Pol B DNA-Polymeraseaktivität und die 3'-5'-Exonukleaseaktivität durch Inkubation der thermostabilen Pol B DNA-Polymerase bei einer erhöhten Temperatur, die > 50 °C ist, wiederherstellbar sind,
und wobei die Pol B DNA-Polymerase ein Fusionsprotein ist, das Pfu, KOD, Tli oder Pfx, oder eine DNA-Polymerase, die unter dem Handelsnamen Vent®, Deep Vene®, Phusion™, oder iProof™, oder einem Fragment davon, das DNA-Polymeraseaktivität und 3'-5'-Exonukleaseaktivität und ein Nukleinsäure-bindendes Polypeptid hat, umfasst.

2. Die modifizierte thermostabile Pol B DNA-Polymerase von Anspruch 1, wobei die Pol B DNA-Polymerase 10His-Pfu-Pae3192 ist.

3. Die modifizierte thermostabile Pol B DNA-Polymerase entsprechend Anspruch 1, wobei das modifizierende Reagenz ausgewählt ist aus der Gruppe, bestehend aus Maleinsäureanhydrid, ein substituiertes Maleinsäureanhydrid, Citraconsäureanhydrid, cis-Aconitsäureanhydrid, 2,3- Dimethylmaleinsäureanhydrid, exo-cis-3,6-endoxo-δ4-Tetrahydrophthalsäureanhydrid; oder 3,4,5, 6-Tetrahydrophthalsäureanhydrid.

4. Die modifizierte thermostabile Pol B DNA-Polymerase von Anspruch 1, wobei die erhöhte Temperatur ≥ 95 °C, ≥ 96,5 °C, oder ≥ 98 °C ist.

5. Die modifizierte thermostabile Pol B DNA-Polymerase von Anspruch 4, wobei die DNA-Polymeraseaktivität und 3'-5'-Exonukleaseaktivität durch Inkubation der thermostabilen Pol B DNA-Polymerase in einem Reaktionspuffer, der einen pH zwischen 7,5 und 8,5 hat, wiederhergestellt wurden.

6. Die thermostabile Pol B DNA-Polymerase entsprechend Anspruch 1, die in der Lage ist, ein detektierbares Amplifikationsprodukt in einer Polymerase-Kettenreaktion herzustellen nach Inkubation bei einer Temperatur, die größer ist als 50 °C, für eine Zeit, die hinreichend ist, um die Pol B DNA-Polymerase zu reaktivieren.

7. Ein Polymerase-Kettenreaktion-Amplifikations-Reaktionsgemisch, umfassend mindestens einen Primer und eine modifizierte Pol B DNA-Polymerase von Anspruch 1.

8. Ein Kit, umfassend eine thermostabile Pol B DNA-Polymerase von Anspruch 1 und einen geeigneter Behälter.

9. Ein Verfahren zur Modifikation einer thermostabilen Pol B DNA-Polymerase, das Verfahren umfassend Reaktion einer thermostabilen Pol B DNA-Polymerase und einem modifizierenden Reagenz der Formel I 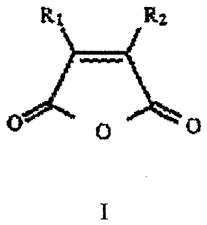 wobei R₁ und R₂ Wasserstoff oder ein C₁-C₄ Alkyl, die verknüpft sein können, sind, und wobei die Reaktion in der Inaktivierung der thermostabilen Pol B DNA-Polymeraseaktivität und der 3'-5'-Exonukleaseaktivität resultiert, wobei die thermostabile Pol B DNA-Polymeraseaktivität und die 3'-5'-Exonukleaseaktivität wiederherstellbar sind, wie in Anspruch 1 wiedergegeben, und wobei die thermostabile Pol B DNA-Polymerase eine Pol B DNA-Polymerase ist, wie in Anspruch 1 wiedergegeben.

10. Ein Verfahren zur Amplifikation einer Zielnukleinsäure, die in einer Probe vorhanden ist, umfassend die Schritte:
a) Kontaktieren der Probe mit einem Amplifikations-Reaktionsgemisch, das einen Primer, der komplementär zu der Zielnukleinsäure ist, und eine modifizierte thermostabile Pol B DNA-Polymerase entsprechend Anspruch 1 enthält; und
b) Inkubieren des resultierenden Gemisches aus Schritt (a) bei einer Temperatur, die größer ist als etwa 50°C, für eine Zeit, die hinreichend ist, um die Pol B DNA-Polymerase zu reaktivieren und die Bildung von Primer-Verlängerungsprodukten gewährt.

11. Das Verfahren von Anspruch 10, wobei jene Amplifikation eine Polymerase-Kettenreaktion ist.

## Revendications

1. ADN polymérase Pol B thermostable modifiée possédant à la fois une activité ADN polymérase et une activité 3'-5' exonucléase, l'ADN polymérase Pol B thermostable modifiée étant produite par la réaction, dans des conditions essentiellement aqueuses, d'une ADN polymérase Pol B thermostable avec un réactif modificateur de formule I 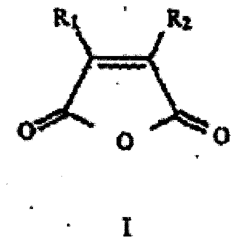 dans laquelle R₁ et R₂ représentent un atome d'hydrogène ou un groupe alkyle en C₁ à C₄ qui peuvent être liés,
où la réaction entraîne l'inactivation de l'activité ADN polymérase Pol B thermostable et de l'activité 3'-5' exonucléase,
où un rapport entre l'activité polymérase et l'activité 3'-5' exonucléase est restaurable en une valeur qui est essentiellement la même qu'avant la modification,
où l'activité ADN polymérase Pol B thermostable et l'activité 3'-5' exonucléase sont restaurables par incubation de l'ADN polymérase Pol B thermostable à une température élevée qui est > 50 °C,
et où l'ADN polymérase Pol B est une protéine de fusion qui comprend Pfu, KOD, TIi, ou Pfx, ou une ADN polymérase vendue sous les noms commerciaux de Vent@, Deep Vent@, Phusion™, ou iProof™, ou l'un de leurs fragments possédant une activité ADN polymérase et une activité 3'-5' exonucléase et un polypeptide liant un acide nucléique.

2. ADN polymérase Pol B thermostable modifiée selon la revendication 1, où l'ADN polymérase Pol B est 10His-Pfu-Pae3192.

3. ADN polymérase Pol B thermostable modifiée selon la revendication 1, où le réactif modificateur est choisi dans le groupe constitué de l'anhydride maléique, un anhydride maléique substitué, l'anhydride citraconique, l'anhydride cis-aconitique, l'anhydride 2,3-diméthylmaléique, l'anhydride exo-cis-3,6-endoxo-δ4-tétrahydrophtalique, ou l'anhydride 3,4,5,6-tétrahydrophtalique.

4. ADN polymérase Pol B thermostable modifiée selon la revendication 1, où la température élevée est ≥ 95 °C, ≥ 96,5 °C, ou ≥ 98 °C.

5. ADN polymérase Pol B thermostable modifiée selon la revendication 4, où l'activité polymérase et l'activité 3'-5' exonucléase ont été restaurées par incubation de l'ADN polymérase Pol B thermostable dans un tampon de réaction ayant un pH situé entre 7,5 et 8, 5.

6. ADN polymérase Pol B thermostable modifiée selon la revendication 1, qui est capable de produire un produit d'amplification détectable dans une réaction en chaîne de la polymérase après incubation à une température qui est supérieure à 50 °C pendant un temps suffisant pour réactiver l'ADN polymérase Pol B.

7. Mélange de réaction d'amplification de la réaction en chaîne de la polymérase, comprenant au moins une amorce et une ADN polymérase Pol B thermostable modifiée selon la revendication 1.

8. Kit comprenant une ADN polymérase Pol B thermostable modifiée selon la revendication 1, et un récipient approprié.

9. Procédé de modification d'une ADN polymérase Pol B thermostable, le procédé comprenant la réaction d'une ADN polymérase Pol B thermostable et d'un réactif modificateur de formule I 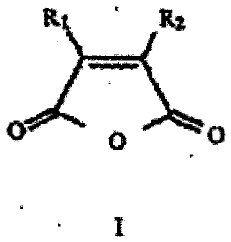 dans laquelle R₁ et R₂ représentent un atome d'hydrogène ou un groupe alkyle en C₁ à C₄ qui peuvent être liés, et où la réaction entraîne l'inactivation de l'activité ADN polymérase Pol B thermostable et de l'activité 3'-5' exonucléase, où l'activité ADN polymérase Pol B thermostable et l'activité 3'-5' exonucléase sont restaurables comme cité dans la revendication 1, et où l'ADN polymérase Pol B est une ADN polymérase Pol B citée dans la revendication 1.

10. Procédé d'amplification d'un acide nucléique cible contenu dans un échantillon comprenant les étapes suivantes :
a) la mise en contact de l'échantillon avec un mélange de réaction d'amplification contenant une amorce complémentaire de l'acide nucléique cible et une ADN polymérase Pol B thermostable modifiée selon la revendication 1 ; et
b) l'incubation du mélange résultant de l'étape (a) à une température qui est supérieure à environ 50 °C pendant un temps suffisant pour réactiver l'ADN polymérase Pol B et permettre la formation de produits d'extension d'amorce.

11. Procédé selon la revendication 10, dans lequel ladite amplification est une réaction en chaîne de la polymérase.
